# EUROPEAN PATENT SPECIFICATION

(11) **EP 2 886 639 B1**
(45) Date of publication and mention of the grant of the patent: **05.12.2018**
(21) Application number: 14189044.2
(22) Date of filing: 15.10.2014
(51) Int. Cl.: C12M 1/107, C12M 1/02, C12M 1/34, C12M 1/38

(54) **A plant for production of biogas**
Anlage zur Herstellung von Biogas
Installation de production de biogaz

(30) Priority: 18.12.2013 SE 1351522
(43) Date of publication of application: 24.06.2015
(73) Proprietor: Methavos SA, 57600 Morsbach (FR)
(72) Inventor: SCHMUTZ, Urs, 793 92 Leksand (SE)
(74) Representative: Bjerkéns Patentbyrå KB (Gävle)

(56) References cited:
- EP-A1- 1 524 315
- EP-A2- 0 617 120
- CN-U- 201 801 518
- CN-U- 202 576 427

## Description

### TECHNICAL FIELD OF THE INVENTION

The present invention relates to a plant for biological degradation of organic material through dry fermentation for production of biogas, which comprises:
- a closed container for the fermentation having in a first end thereof at least one opening for feeding in organic material and in a second end opposite to said first end at least one opening for discharging fermented material,
- one or several openings for removal of biogas produced in the container in the upper part of the container,
- members configured to be driven to stir organic material inside the container fed from the first to the second end of the container, and
- members configured to be controllable to generate heat for heating the organic material in the container,
wherein the plant further comprises at least three zones arranged in a row between said first and second end of the container to be consecutively passed by biologic material fed through the container, each zone having at least one said stirring member, the stirring member of each zone being controllable separately from the stirring members belonging to the other zones.

"Dry fermentation" is here to be interpreted as a fermentation of organic material in the form of viscous biomass having a dry content in the order of 20-50%, mostly 30-35%.

The organic material may be of any kind suitable to be degraded by influence of microorganisms through fermentation while producing biogas, such as for instance garden waste, domestic waste and animal faeces. The biogas produced may be used for example for driving gas engines for generating electric current or as fuel in vehicles or for heating.

The fermentation of the organic material occasionally contained in said container is most efficient at a certain temperature, at which the microorganisms in the material are most active. It is for that reason desired to try to keep the temperature of the material close to such a temperature. The plant has for that sake said heating members which may ensure that the temperature of the material is raised if it is too low. The temperature may at the same time be influenced in the direction of falling by activating the stirring members. The "work" of the microorganisms also creates heat energy, and there may be a need to conduct this energy away for preventing that the temperature gets too high. The stirring is also important for increasing the reactions between microorganisms in the material and by that speed up the production of biogas.

The duration of stay of the organic material in the container of a plant mentioned above before it is judged as completely fermented and is ready to be discharged from the container is typically in the order of 5-25 days. It would of course from a cost point of view be advantageous if this duration of stay may be shortened, so that more biogas may be produced per time unit for a predetermined size of the container.

### BACKGROUND ART

A plant of the type defined in the introduction is already known from documents EP0617120 A2 and CN201801518 U. Even if the plants disclosed in these documents function there is of course a desire to improve the function of such plants.

### SUMMARY OF THE INVENTION

The object of the present invention is to provide a plant of the type defined in the introduction, which is improved in at least some aspect with respect to such plants already known.

Accordingly, the present invention pertains to a plant for biological degradation of organic material through dry fermentation for production of biogas, which comprises:
▪ a closed container (1) for the fermentation having in a first end (2) thereof at least one opening (3) for feeding in organic material and in a second end (4) opposite to said first end at least one opening (5) for discharging fermented material,
▪ one or several openings (7) for removal of biogas produced in the container in the upper part of the container,
▪ members (29-38) configured to be driven to stir organic material inside the container fed from the first to the second end of the container, and
▪ members (17, 18, 21, 22) configured to be controllable to generate heat for heating the organic material in the container,
wherein the plant further comprises at least three zones (24-28) arranged in a row between said first and second end of the container to be consecutively passed by biologic material fed through the container, each zone having at least one said stirring member (29-38), the stirring member of each zone being controllable separately from the stirring members belonging to the other zones,
characterized in that that the plant comprises a control unit (16) configured to control stirring, temperature and duration of stay of organic material present in each zone separately for each zone by controlling said stirring members,
that the plant comprises means (90) for measuring the temperature of the organic material, preferably in connection to each zone, and that the control unit (16) is configured to control the speed of the respective stirring member (29-38) and said heating members (17, 18, 21, 22) in dependence upon the temperature measured so as to maintain the temperature of the organic material within a determined reference value interval.

By dividing the container into at least three zones in a row and the stirring members of each zone are controllable independently of the stirring members belonging to the other zones stirring, temperature and duration of stay of organic material present in each zone may be controlled separately, so that an optimum of the efficiency of the biogas production may be achieved in each part (zone) of the container. The temperature may in this way in each zone be maintained on a desired level by controlling the stirring. The heating through the heating members and this stirring may be adapted to each other for obtaining an optimum result, so that the total duration of stay of the organic material in the container may by that be optimized, i.e. shortened.

According to an embodiment of the invention the stirring members are configured to be activated so as to move heat energy through organic material mass present in the container upwardly through said openings for removal of biogas. The temperature above the surface of the organic material will be lower than the temperature of the organic material and heat energy will for that sake be transported through the organic material upwardly to the surface and emitted there. This transport may be facilitated and promoted the more the more intense the stirring is and an increased stirring will by that act towards a lower temperature of the organic material. Accordingly, heat energy may through stirring in each zone efficiently be removed by making it to rise and warm air or biogas to leave the container through said openings, so that the temperature of the organic material is then lowered, at the same time as the reaction speed of the microorganisms in the material is increased by enabling them to contact more "neighbours". Would then the temperature have a tendency to sink below the desired temperature this may at the same time be counteracted by control of the heating members.

According to another embodiment of the invention each zone has at least two stirring members located side by side as seen in the feeding direction from said first to said second end so that at least two rows of stirring members are arranged between said first and second end of the container. By such an arrangement of several stirring members side by side in each zone the possibility to influence the duration of stay of the organic material in the respective zone through control of the stirring members is improved, and this possibility gets particularly good when according to another embodiment of the invention the control unit is configured to control stirring members belonging to one and the same zone independently of each other. It gets by this possible to control adjacent stirring members so that they co-operate so as to feed the organic material towards the following zone or counteracting such feeding and prolong the duration of stay of the organic material in the zone through stirring.

According to another embodiment of the invention said stirring members are controllable to move around substantially vertical or vertical rotation axles. An advantageous stirring and movement of the organic material inside the container may by this be efficiently obtained.

According to another embodiment of the invention the rotation direction of the stirring members is reversible, and the control unit is configured to control the rotation direction of the stirring members belonging to the same zone independently of each other. The duration of stay of the organic material in the zone may by this for example be prolonged by bringing adjacent stirring members to rotate in opposite directions.

According to another embodiment of the invention the plant comprises means for at least partially delimiting each of said zones with respect to adjacent zones. The duration of stay of the organic material in each zone may be influenced by the arrangement of such means. According to an embodiment of the invention said means comprises partition walls configured to delimit consecutive said zones, and the partition walls are designed to allow feeding of organic material from one zone to another by either having one or several openings or being entirely or partially removable from a position in which they separate adjacent zones. A distinct dividing of the container into separate zones in which duration of stay and temperature of the organic material may be controlled substantially independently of events in other zones of the container is by that achieved. It is then according to another embodiment of the invention advantageous to have the partition walls designed to form openings between adjacent zones with a total cross-section of 30%-80%, 40%-60%, 50%-70%, 50%-60% or about 50% of the cross-section of the container perpendicularly to the feeding direction from the first to the second end. Such an open degree, especially of about 50%, of the partition walls has turned out to result in an efficient restriction of adjacent zones and nevertheless a possibility to obtain a sufficiently efficient feeding of the organic material at a desired speed from one zone to another.

According to another embodiment of the invention said means comprises objects with the pyramidal shape arranged on the bottom of the container with a pyramid tip pointing upwardly. Rather dense mass reaching said objects with pyramidal shape may then slide down on these objects and back into the zone.

According to another embodiment of the invention said stirring members comprise blade-like elements attached on different levels to an axle controllable by said control unit to rotate. Organic material in different depths in the container may by this be efficiently stirred for favourable influence upon the degradation by the microorganisms and the production of biogas. According to another embodiment of the invention at least some, for example the uppermost blade-like elements on each axle, of the blade-like elements are adjustable in different inclinations for forming differently large projection surfaces in the direction in which they move when the axle rotates. The influence of such blade-like elements upon the organic material when stirring this material may by that be adjusted, i.e. the flowing direction generated when they rotate may be varied. It may in particular by a change of the inclination of the uppermost blade-like elements be ensured that the upper surface layer of the organic material in the container is broken so that bubbles of biogas travelling upwardly may easier penetrate through this uppermost layer. It may by that also be prevented that the gas bubbles lift the upper layer so that this is pressed into the openings for removal of biogas and obstruct the conduits connected thereto.

According to another embodiment of the invention each said axle is at the lower end thereof provided with at least one blade-like element configured to, upon rotation of the axle, move like a rake along a bottom of the container so as to move solid particles, such as gravel and stones, contained in said organic material and deposited on this bottom. It may by this efficiently be ensured that such material will not remain on the bottom of the container and be deposited there for finally disturbing the entire function of the plant, but such "waste" may instead efficiently be fed forwardly through the container along the bottom thereof for then being removed in a suitable manner from the container in said second end. According to a further development of the embodiment last mentioned each said axle is designed to in a rest position extend from the axle radially further than the shortest distance from the axle to pieces defining said area and foldable so as to when hitting a said piece through rotation of the axle be folded while storing potential energy and through the extreme end thereof move along said piece so as to return towards the rest position when said distance to said piece increases. By such a length of the blade-like element in said rest position and foldability thereof while storing potential energy said solid particles deposited on the bottom of the container may be reached over substantially the entire area of each zone and removed over said bottom of the container towards the second end.

According to the invention the plant comprises means for measuring the temperature of the organic material, preferably in connection to each zone, and the control unit is configured to control the speed of the respective stirring member as well as said heating members in dependence upon the temperature measured so as to maintain the temperature of the organic material within a predetermined reference value interval. The control unit is according to an embodiment of the invention designed to control the stirring members and the heating members so as to keep the temperature of the organic material between 50°C and 60°C, between 53°C and 57°C or at approximately 55°C, which are suitable temperatures pleasant for the microorganisms, so that they may efficiently degrade the organic material through fermentation.

According to another embodiment of the invention the plant has means for recirculating a part of the fermented material reaching said second end of the container to said first end for feeding it into the container at this end again, said recirculated part being for instance 1-10% or 2-6% of the fermented material reaching said second end. By such a recirculation the concentration of bacteria of the organic material present in the zone being the first one as seen in the feeding direction through the container is increased, which accelerates starting of the production of biogas from the organic material which has been present the shortest time in the container and which has the highest concentration of still not fermented material.

According to another embodiment of the invention the plant comprises means for controlling the average speed of the movement of the organic material between said first and second end of the container by controlling the speed of feeding organic material into the container.

According to another embodiment of the invention the plant comprises means for supplying organic material having a dry content of 20%-40%, 25%-35% or 30%-35% to the container, which are suitable dry contents for biological degradable material for production of biogas in a plant of the type according to the invention.

Further advantages as well as advantageous features of the invention appear from the following description.

### BREIF DESCRIPTION OF THE DRAWINGS

With reference to the appended drawings, below follows a description of embodiments of the invention cited as examples.

In the drawings:
- Fig 1: is a sectioned side view of a plant according to a first embodiment of the invention,
- Fig 2: is a view from above of the plant shown in Fig 1,
- Fig 3: is a view sectioned according to C-C in Fig 1 of the plant in Fig 1,
- Fig 4: is a simplified, partially sectioned view of a part of the plant according the invention,
- Fig 5: is a simplified view from above of a part of the plant in Fig 1,
- Fig 6: is a view corresponding to Fig 1 but simplified with respect thereto of a plant according to a second embodiment of the invention,
- Fig 7: is a simplified view from above of the plant according to Fig 6, and
- Fig 8: is a simplified view corresponding to Fig 3 of the plant in Fig 6.

### DETAILED DESCRIPTION OF EMBODIMENTS OF THE INVENTION

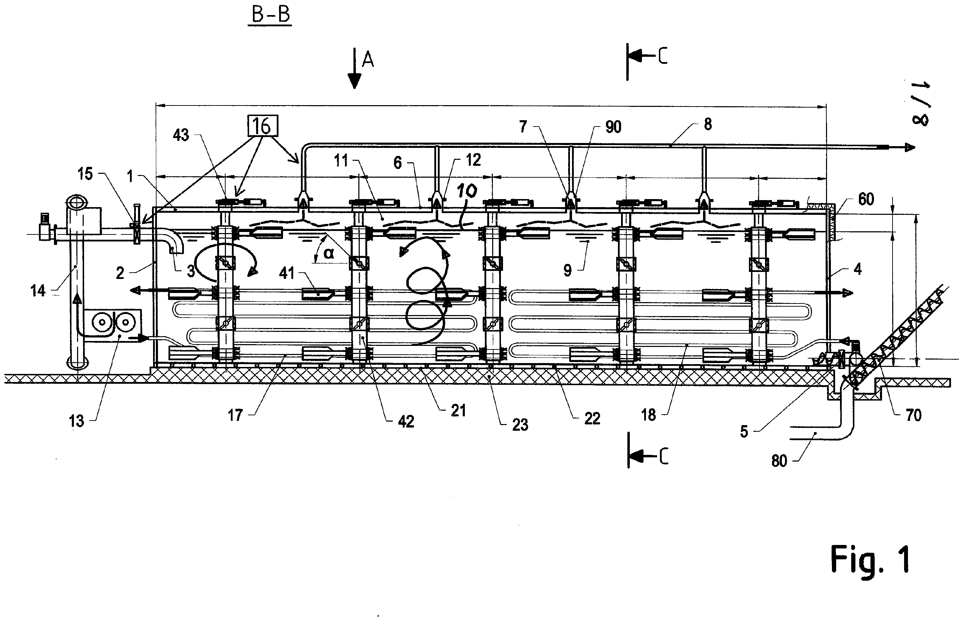

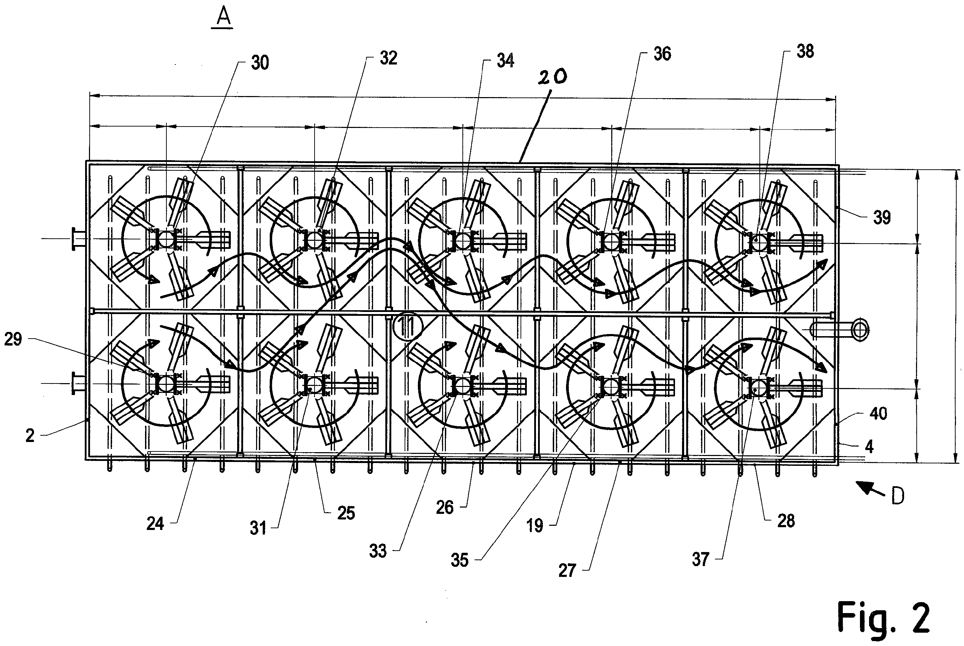

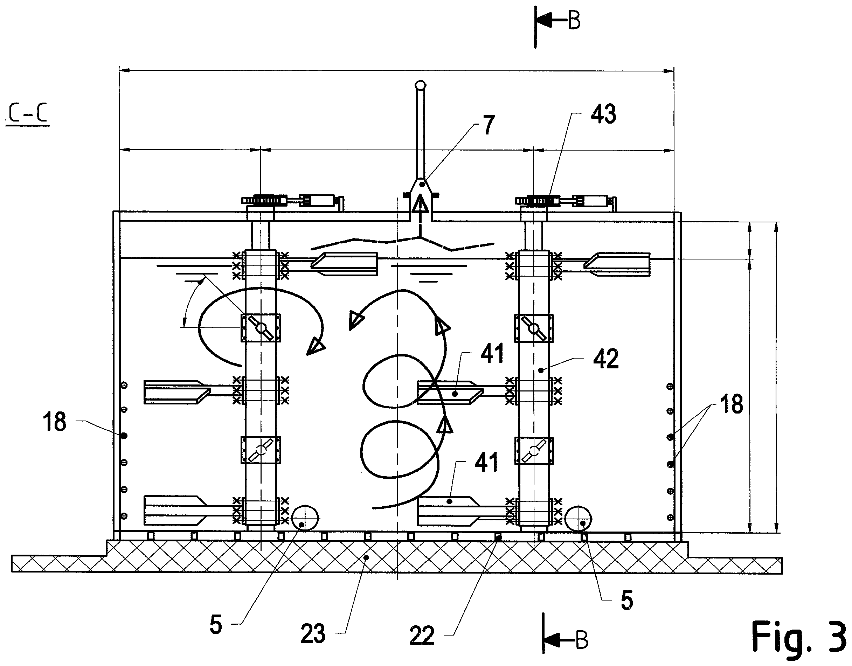

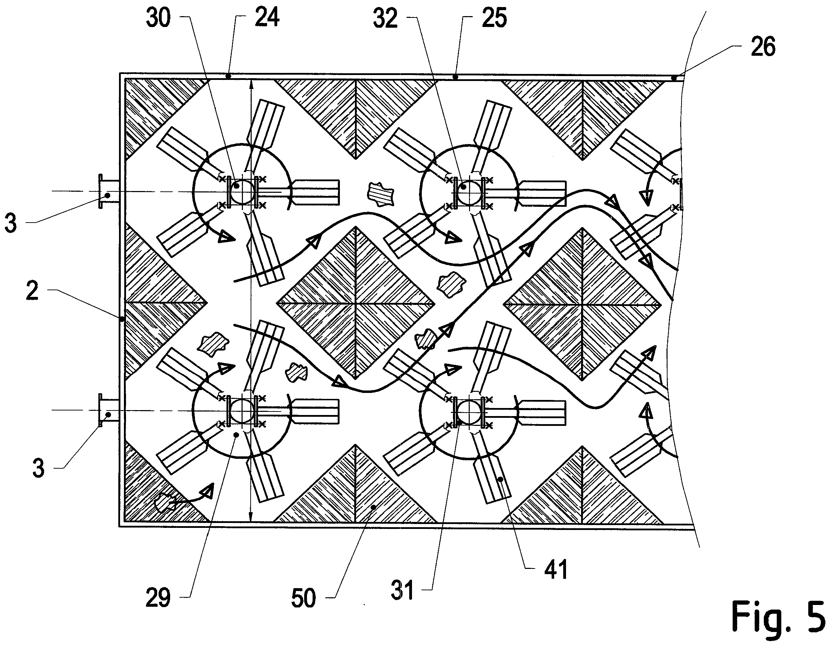

Fig 1 illustrates the construction of a plant according to a first embodiment of the invention for biological degradation of organic material through dry fermentation for production of biogas, and the construction and function of this plant will now be explained while making reference also to Figs 2-5.

The plant has an elongated closed container 1 which may for instance have a length of 20 metres, a width of 6 metres and a height of 5 metres (the container has typically a length being about three times the width thereof), and this container has at a first end 2 at least one opening 3 for feeding in organic material in the form of a viscous bio mass with a dry content of for example 30-35% and at a second end 4 in the longitudinal direction of the container opposite to the first end at least one opening 5 for discharging fermented material. In the upper part of the container, more exactly in the ceiling 6 thereof, there are a plurality, in this case four, openings 7, which are connected to a conduit 8 for removing biogas produced in the container, leaving the viscous bio mass 9 at the upper surface 10 thereof and entering into the gas space 11 in the upper part of the container, which is indicated through the arrows 12.

The organic material is supplied to the plant through an arrangement 13 designed to crush the organic material to only contain parts with dimensions below a determined value, such as for example 40 mm, so as to avoid that large lumps that may disturb the biological process in the container enter the container. A device 14 for feeding the organic material into the container through the opening 3 follows upon the crushing arrangement 13. A blocking member 15 is controllable through a control unit 16 for influencing the speed by which the organic material is fed into the container.

The plant has members configured to be controllable to generate heat for heating the organic material in the container, and in this embodiment of the invention there are more exactly six such heating members arranged in the form of two separate heating loops 17, 18 each along a half of each longitudinal side wall 19, 20 of the container 1 and two heating loops 21, 22 each covering a half of the bottom 23 of the container. The different heating members are advantageously controllable independently of each other through the control unit 16.

The container has five zones 24-28 in a row between the first 2 and second end 4 to be consecutively passed by biological material fed through the container, and each zone has two stirring members 29-38 located side by side as seen in the direction of feeding from the first to the second end, so that two rows 39, 40 of stirring members are arranged between the first and second end of the container.

The stirring members comprises blade-like wing- or paddle-like elements 41 arranged on different levels along a substantially vertical axle 42 which is rotatably arranged in bearings. Driving members 43 in the form of suitable motors are arranged for each stirrring member for rotating this axle. The control unit 16 is configured to control these driving members 43 and by that the movements of the stirring members completely independently of each other, in which also the rotation direction of the stirring members may be reversed when desired.

The blade-like elements of the stirring members have an inclination indicated by the angle α in Fig 1 so as to form a certain projection surface in the direction of movement of the element when stirring. This angle is advantageously adjustable for the uppermost blade-like element of each stirring member so that this may efficiently be influenced to break the upper surface layer of the biomass 9 so that biogas produced within the biomass may smoothly leave the biomass. Means in the form of objects 50 with a pyramidal shape are arranged on the bottom of the container with a pyramid tip pointing upwardly so as to at least partially delimit each of the zones with respect to adjacent zones. Other type of delimiting members, such as any type of removable partition walls, are also conceivable.

It appears at 60 in Fig 1 that the container has a surrounding heat insulating layer and a member 70 for feeding fermented material discharged through the opening 5 away as well as means 80 for recirculating a part, such as 1-10%, of the fermented material to the device 14 for feeding it into the container at the first end 2 thereof so as to increase the bacteria content in the organic material in the zone 24 for rapidly starting the degradation of the organic material there.

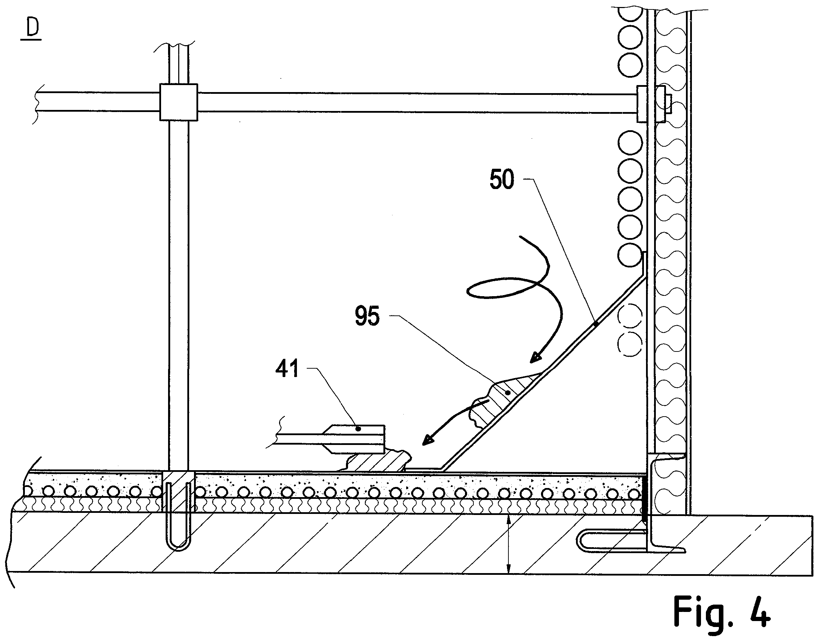

The function of the plant according to the invention is as follows. Organic material, such as garden waste, domestic waste and animal faeces is supplied to the container through the opening 3 at one end 2 thereof and the material moves slowly, such as during a period of time in the order of 20 days to the opposite, second end 4 during fermentation of the organic material and production of biogas. It is then important to keep the temperature of the biomass at the level, normally about 55°C, at which the microorganisms in this mass thrives. The stirring of the biomass does then also promote the reactions for degradation thereof. Temperature sensors are advantageously arranged in connection to each zone, and the control unit 16 is configured to control the stirring members and the heating members in dependence of information from these temperature sensors so as to keep the temperature of the biomass at a suitable level. By the fact that the stirring members in each zone may be controlled separately it gets possible to create optimum conditions with respect to temperature, stirring and duration of stay in each zone. Is there for instance a wish to have the organic material to stay extra-long in one zone the two stirring members of that zone may be brought to rotate in opposite directions, whereas they may be brought to rotate in the same direction in the case of a desire to influence the biomass in the feeding direction forwardly. A mixture of the biomass with on one hand release of gas and on the other distribution of bacteria as a result also takes place through the stirring. It is in the figures through arrows shown how the organic material moves in the container upon rotation of the stirring members. The stirring members may alternatively be rotated and stand still. It is in Fig 4 illustrated how more dense biomass 95 reaching an element 50 with pyramidal shape may move downwardly along this so as to be guided into the zone in question again.

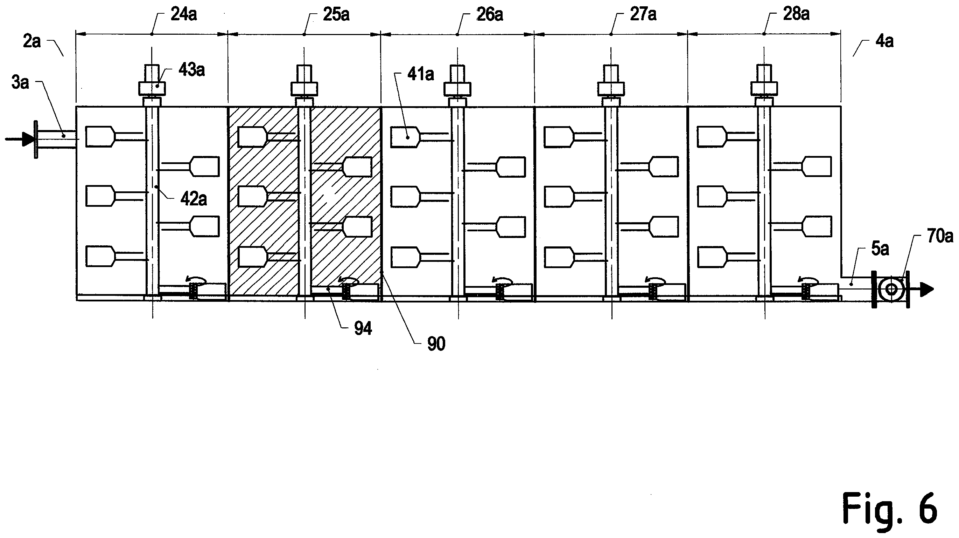

The construction of a plant according to a second embodiment of the invention is very simplifiedly illustrated in Fig 6-8, and a number of components present in that plant and shown for the plant according to the first embodiment of the invention are omitted and only the differences of this embodiment with respect to the first one are illustrated. The components shown for the plant according to this second embodiment of the invention and corresponding to components of the first embodiment are provided with the same reference numerals with the addition of an a. Consecutive zones 24a-28a of this second embodiment of the invention are partially delimited with respect to each other through stationary partition walls 90, which are each formed by two triangular disc-like elements 91, 92, for example of sheet metal, which together covers 50% of the cross-section of the container as seen in the feeding direction from the first end 2a to the second end 4a. The disc-like elements have the shape of equally sided triangles with a base extending along the respective side wall 19a, 20a of the container and the tips meeting each other in the midpoint 93 of the container with respect to the cross-section thereof. It has turned out that such a delimitation of adjacent zones results in a good possibility to control the duration of stay and the treatment of the organic material without any remarkable influence upon what happens in the adjacent zone and still ensure that the material may be fed with a desired speed from one zone to the other without a prolonged or shortened duration of stay in the zone of any material coming into any particular part of the zone with respect to other material. It is for that sake advantageous that the partition wall 90 leaves the entire or substantially the entire region closest to the bottom 23a of the container free for passage of organic material from one zone to the other.

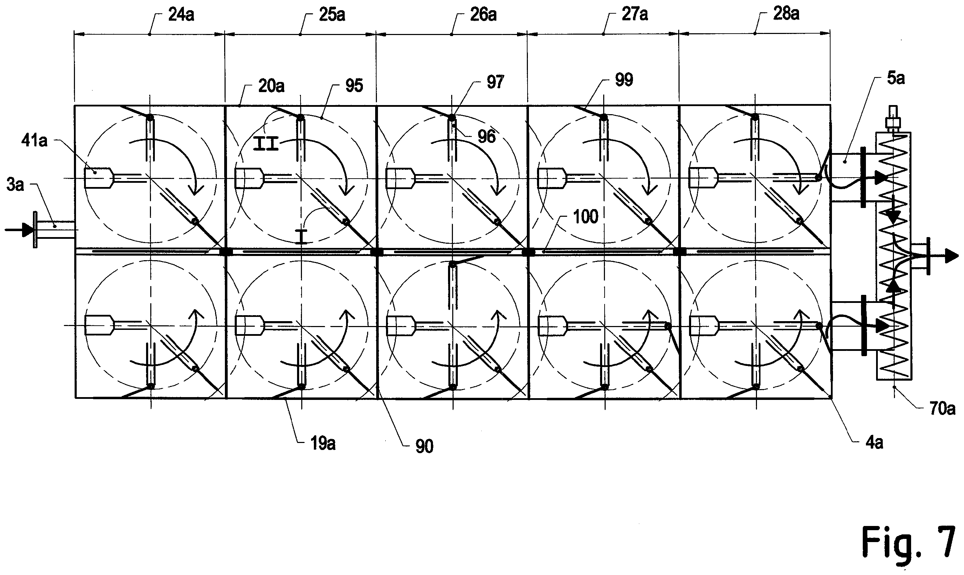

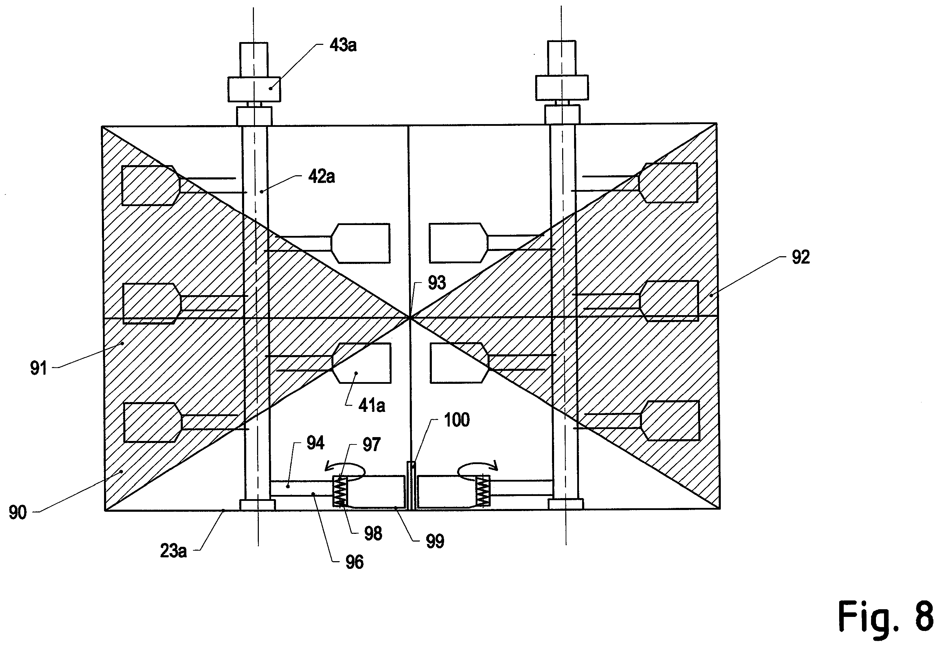

Another difference of the plant according to the second embodiment of the invention with respect to the first one is that each axle 42a is at the lower end provided with at least one blade-like element 94, as seen in the direction of extension of the axle 42a with the axle arranged in the midpoint of a rectangular, here square, area 95 designed in a rest position I (see Fig 7) to extend from the axle radially longer than the shortest distance from this axle to said pieces defining said area. Each such lowermost blade-like element 94 is designed foldable so as to when hitting a said piece, such as a longitudinal sidewall 19a, 20a, during rotation of the axle 42a be folded while storing potential energy and through the extreme end thereof move along that piece and return towards the rest position when the distance to that piece increases. This foldability is realized by constructing the blade-like element 94 of two separate parts with a first rod element 96 rigidly connected to the axle 42a and a rake or scraper element 99 connected thereto through a hinge 97 loaded with a spring member 98. The spring member 98 will then in an unloaded position I of the rake element bring this to extend in the prolongation of the rod 96, while it in the position according to II in Fig 7 may be folded with respect thereto. It appears from Figs 7 and 8 how adjacent said areas in the same zone are at the bottom delimited with respect to each other through a partition beam 100.

The lowermost blade-like element 94 of each axle 42a will through the rake element 99 thereof upon rotation of the axle move like a rake along the bottom 23a of the container for moving solid particles deposited on this bottom, such as gravel and stones, which are present in the organic material. Such sediment will by this efficiently be scraped away from the bottom of the container and in the second end 4a of the container be discharged therefrom and moved away through the feeding member 70a.

The invention is of course not in any way restricted to the embodiments described above, but many possibilities to modifications thereof would be apparent to a person skilled in the art without departing from the scope of the invention as defined in the appended claims.

The foldability of said lowermost blade-like elements may for example be realized by making the whole or a part of the element flexible.

## Claims

1. A plant for biological degradation of organic material through dry fermentation for production of biogas, which comprises:
• a closed container (1) for the fermentation having in a first end (2) thereof at least one opening (3) for feeding in organic material and in a second end (4) opposite to said first end at least one opening (5) for discharging fermented material,
• one or several openings (7) for removal of biogas produced in the container in the upper part of the container,
• members (29-38) configured to be driven to stir organic material inside the container fed from the first to the second end of the container, and
• members (17, 18, 21, 22) configured to be controllable to generate heat for heating the organic material in the container,
wherein the plant further comprises at least three zones (24-28) arranged in a row between said first and second end of the container to be consecutively passed by biologic material fed through the container, each zone having at least one said stirring member (29-38), the stirring member of each zone being controllable separately from the stirring members belonging to the other zones, **characterized in that** that the plant comprises a control unit (16) configured to control stirring, temperature and duration of stay of organic material present in each zone separately for each zone by controlling said stirring members, that the plant comprises means (90) for measuring the temperature of the organic material, preferably in connection to each zone, and that the control unit (16) is configured to control the speed of the respective stirring member (29-38) and said heating members (17, 18, 21, 22) in dependence upon the temperature measured so as to maintain the temperature of the organic material within a determined reference value interval.

2. A plant according to claim 1, **characterized in that** the control unit (16) is configured to control the stirring member (29-38) and the heating members (17, 18, 21, 22) so as to maintain the temperature of the organic material between 50°C and 60°C, between 53°C and 57°C or at approximately 55°C.

3. A plant according to claim 1 or 2, **characterized in that** the stirring members (29-38) are configured to be activated so as to move heat energy through organic material mass present in the container upwardly through said openings (7) for removal of biogas.

4. A plant according to any of the preceding claims, **characterized in that** each zone (24-28) has at least two stirring members (29-38) located side by side as seen in the feeding direction from said first to said second end so that at least two rows (39, 40) of stirring members are arranged between said first (2) and second (4) end of the container.

5. A plant according to claim 4, **characterized in that** the control unit (16) is configured to control said stirring members belonging to one and the same zone independently of each other.

6. A plant according to any of the preceding claims, **characterized in that** said stirring members are controllable to move around substantially vertical or vertical rotation axles (42).

7. A plant according to claim 4 or 5 and claim 6, **characterized in that** the rotation direction of the stirring members (29-38) is reversible, and that the control unit (16) is configured to control the rotation direction of the stirring members belonging to the same zone independently of each other.

8. A plant according to any of the preceding claims, **characterized in that** it comprises means (50) for at least partially delimiting each of said zones (24-28) with respect to adjacent zones.

9. A plant according to claim 8, **characterized in that** said means comprises partition walls (90) configured to delimit consecutive said zones (24a-28a), and that the partition walls are designed to allow feeding of organic material from one zone to another by either having one or several openings or being entirely or partially removable from a position in which they separate adjacent zones.

10. A plant according to claim 9, **characterized in that** the partition walls (90) are designed to form openings between adjacent zones with a total cross-section of 30%-80%, 40%-60%, 50%-70%, 50%-60% or about 50% of the cross-section of the container perpendicularly to the feeding direction from the first (2a) to the second (4a) end.

11. A plant according to any of the preceding claims, **characterized in that** said stirring members (29-38) comprise blade-like elements (41) attached on different levels to an axle (42) controllable by said control unit (16) to rotate.

12. A plant according to claim 11, **characterized in that** each said axle (42a) is at the lower end provided with at least one blade-like element (94) configured to, upon rotation of the axle, move like a rake along a bottom (23a) of the container so as to move solid particles, such as gravel and stones, contained in said organic material and deposited on this bottom.

13. A plant according to claim 12, **characterized in that** each said axle (42a) is arranged in a midpoint of a rectangular, such as square, area (95) as seen in the direction of extension of said axle, and that said blade-like element (94) at the lower end of the axle is designed to in a rest position extend from the axle (42a) radially further than the shortest distance from the axle to pieces (100) defining said area and foldable so as to when hitting a said piece through rotation of the axle be folded while storing potential energy and through the extreme end thereof move along said piece so as to return towards the rest position when said distance to said piece increases.

14. A plant according to any of the preceding claims, **characterized in that** it comprises means (80) for recirculating a part of the fermented material reaching said second end (4) of the container (1) to said first end (2) for feeding it into the container at this end again, said recirculated part being for instance 1-10% or 2-6% of the fermented material reaching said second end.

15. A plant according to any of the preceding claims, **characterized in that** it comprises means (14) for supplying organic material with a dry content of 20%-40%, 25%-35% or 30%-35% to the container.

## Patentansprüche

1. Anlage für einen biologische Abbau von organischem Material durch Trockenfermentierung zur Herstellung von Biogas, das umfasst:
• einen geschlossenen Behälter (1) für die Fermentierung, der in seinem ersten Ende (2) zumindest eine Öffnung (3) zum Zuleiten von organischem Material und in einem zweiten Ende (4), gegenüber dem ersten Ende, zumindest eine Öffnung (5) zum Abgeben von fermentiertem Material aufweist,
• eine oder mehrere Öffnungen (7) zur Entnahme von Biogas, das im Behälter erzeugt wurde, im oberen Teil des Behälters,
• Elemente (29-38), die eingerichtet sind, angetrieben zu werden, um das organische Material im Inneren des Behälters zu rühren, das vom ersten zum zweiten Ende des Behälters geleitet wird, und
• Elemente (17, 18, 21, 22), die eingerichtet sind, steuerbar zu sein, um Wärme zum Erwärmen des organischen Materials im Behälter zu erzeugen,
wobei die Anlage ferner zumindest drei Zonen (24-28) umfasst, die in einer Reihe zwischen dem ersten und zweiten Ende des Behälters angeordnet sind, um der Reihe nach von dem biologischen Material durchlaufen zu werden, das durch den Behälter geleitet wird, wobei jede Zone zumindest ein Rührelement (29-38) umfasst, wobei das Rührelement jeder Zone separat von den Rührelementen steuerbar ist, die zu den anderen Zonen gehören, **dadurch gekennzeichnet, dass** die Anlage eine Steuereinheit (16) umfasst, die eingerichtet ist, Rühren, Temperatur und Verweildauer von organischem Material, das in jeder Zone vorhanden ist, separat für jede Zone durch Steuern der Rührelemente zu steuern, dass die Anlage Mittel (90) zum Messen der Temperatur des organischen Materials umfasst, vorzugsweise in Verbindung mit jeder Zone, und dass die Steuereinheit (16) eingerichtet ist, die Geschwindigkeit des jeweiligen Rührelements (29-38) und der Heizelemente (17, 18, 21, 22) abhängig von der gemessenen Temperatur zu steuern, um so die Temperatur des organischen Materials innerhalb eines bestimmten Referenzwertintervalls zu halten.

2. Anlage nach Anspruch 1, **dadurch gekennzeichnet, dass** die Steuereinheit (16) eingerichtet ist, das Rührelement (29-38) und die Heizelemente (17, 18, 21, 22) so zu steuern, dass die Temperatur des organischen Materials zwischen 50°C und 60°C, zwischen 53°C und 57°C oder bei annähernd 55°C gehalten wird.

3. Anlage nach Anspruch 1 oder 2, **dadurch gekennzeichnet, dass** die Rührelemente (29-38) eingerichtet sind, aktiviert zu werden, um Wärmeenergie durch organische Materialmasse, die im Behälter vorhanden ist, nach oben durch die Öffnungen (7) zur Entfernung von Biogas zu bewegen.

4. Anlage nach einem der vorangehenden Ansprüche, **dadurch gekennzeichnet, dass** jede Zone (24-28) zumindest zwei Rührelemente (29-38) hat, die, bei Betrachtung in der Zuleitungsrichtung vom ersten zum zweiten Ende, Seite an Seite liegen, sodass zumindest zwei Reihen (39, 40) von Rührelementen zwischen dem ersten (2) und zweiten (4) Ende des Behälters angeordnet sind.

5. Anlage nach Anspruch 4, **dadurch gekennzeichnet, dass** die Steuereinheit (16) eingerichtet ist, die Rührelemente, die zu ein und derselben Zone gehören, unabhängig voneinander zu steuern.

6. Anlage nach einem der vorangehenden Ansprüche, **dadurch gekennzeichnet, dass** die Rührelemente steuerbar sind, um sich um im Wesentlichen vertikale oder vertikale Drehachsen (42) zu bewegen.

7. Anlage nach Anspruch 4 oder 5 und Anspruch 6, **dadurch gekennzeichnet, dass** die Drehrichtung der Rührelemente (29-38) reversibel ist und dass die Steuereinheit (16) eingerichtet ist, die Drehrichtung der Rührelemente, die zu derselben Zone gehören, unabhängig voneinander zu steuern.

8. Anlage nach einem der vorangehenden Ansprüche, **dadurch gekennzeichnet, dass** sie Mittel (50) zum zumindest teilweise Abgrenzen jeder der Zonen (24-28) in Bezug auf benachbarte Zonen umfasst.

9. Anlage nach Anspruch 8, **dadurch gekennzeichnet, dass** die Mittel Trennwände (90) umfassen, die eingerichtet sind, aufeinanderfolgende Zonen (24a-28a) abzugrenzen, und dass die Trennwände eingerichtet sind, ein Zuleiten von organischem Material aus einer Zone zu einer anderen zu erlauben, indem entweder eine oder mehrere Öffnungen vorhanden sind, oder sie vollständig oder teilweise aus einer Position entfernbar sind, in der sie benachbarte Zonen trennen.

10. Anlage nach Anspruch 9, **dadurch gekennzeichnet, dass** die Trennwände (90) eingerichtet sind, Öffnungen zwischen benachbarten Zonen mit einem Gesamtquerschnitt von 30%-80%, 40%-60%, 50%-70%, 50%-60% oder etwa 50% des Querschnitts des Behälters senkrecht zur Zuleitungsrichtung vom ersten (2a) zum zweiten (4a) Ende zu bilden.

11. Anlage nach einem der vorangehenden Ansprüche, **dadurch gekennzeichnet, dass** die Rührelemente (29-39) schaufelartige Elemente (41) auf verschiedenen Ebenen zu einer Achse (42) umfassen, die steuerbar durch die Steuereinheit (16) in Drehung versetzt werden kann.

12. Anlage nach Anspruch 11, **dadurch gekennzeichnet, dass** jede Achse (42a) am unteren Ende mit zumindest einem schaufelartigen Element (94) versehen ist, das eingerichtet ist, sich bei Drehung der Achse wie ein Rechen entlang eines Bodens (23a) des Behälters zu bewegen, um so feste Teilchen, wie Kies und Steine, die im organischen Material enthalten sind und sich auf diesem Boden abgesetzt haben, zu bewegen.

13. Anlage nach Anspruch 12, **dadurch gekennzeichnet, dass** jede Achse (42a), betrachtet in der Verlaufsrichtung der Achse, an einem Mittelpunkt einer rechteckigen, wie einer quadratischen, Fläche (95) angeordnet ist und dass das schaufelartige Element (94) am unteren Ende der Achse eingerichtet ist, sich in einer Ruheposition von der Achse (42a) radial weiter als über die kürzeste Distanz von der Achse zu Teilen (100) zu erstrecken, die die Fläche definieren, und faltbar zu sein, sodass es, wenn es während der Drehung der Achse auf das Teil trifft, gefaltet wird, während mögliche Energie gespeichert wird, und sich mit seinem äußersten Ende entlang des Teils bewegt, um so mit zunehmender Distanz zu dem Teil in die Ruheposition zurückzukehren.

14. Anlage nach einem der vorangehenden Ansprüche, **dadurch gekennzeichnet, dass** sie Mittel (80) zum Rezirkulieren eines Teils des fermentierten Materials, das das zweite Ende (4) des Behälters (1) erreicht, zum ersten Ende (2) umfasst, um dieses an diesem Ende erneut in den Behälter zu leiten, wobei der rezirkulierte Teil beispielsweise 1-10% oder 2-6% des fermentierten Materials, das das zweite Ende erreicht.

15. Anlage nach einem der vorangehenden Ansprüche, **dadurch gekennzeichnet, dass** sie Mittel (14) zum Zuleiten von organischem Material mit einem Trockengehalt von 20% - 40%, 25% - 35% oder 30% - 35% zum Behälter umfasst.

## Revendications

1. Installation de dégradation biologique d'une matière organique par fermentation sèche pour une production de biogaz, comprenant :
- un récipient fermé (1) pour la fermentation comportant, à une première extrémité (2) de celui-ci, au moins une ouverture (3) pour l'alimentation de matière organique et, à une deuxième extrémité (4) à l'opposé de ladite première extrémité, au moins une ouverture (5) pour décharger une matière fermentée,
- une ou plusieurs ouvertures (7) pour l'enlèvement du biogaz produit dans le récipient dans la partie supérieure du récipient,
- des organes (29-38) configurés pour être amenés à brasser une matière organique à l'intérieur du récipient, alimentée depuis la première extrémité à la deuxième extrémité du récipient, et
- des organes (17, 18, 21, 22) configurés pour pouvoir être commandés pour générer de la chaleur afin de chauffer la matière organique dans le récipient,
dans laquelle l'installation comprend en outre au moins trois zones (24-28) agencées dans une rangée entre ladite première extrémité et ladite deuxième extrémité du récipient pour être passées consécutivement par une matière biologique alimentée à travers le récipient, chaque zone comportant au moins un dit organe de brassage (29-38), l'organe de brassage de chaque zone pouvant être commandé distinctement des organes de brassage appartenant aux autres zones, **caractérisée en ce que** l'installation comprend une unité de commande (16) configurée pour commander le brassage, la température et la durée de séjour de la matière organique présente dans chaque zone distinctement pour chaque zone en commandant lesdits organes de brassage, **en ce que** l'installation comprend des moyens (90) pour mesurer la température de la matière organique, de préférence en relation avec chaque zone, et **en ce que** l'unité de commande (16) est configurée pour commander la vitesse de l'organe de brassage respectif (29-38) et lesdits organes de chauffage (17, 18, 21, 22) en fonction de la température mesurée de manière à maintenir la température de la matière organique à l'intérieur d'un intervalle de valeurs de référence déterminé.

2. Installation selon la revendication 1, **caractérisée en ce que** l'unité de commande (16) est configurée pour commander l'organe de brassage (29-38) et les organes de chauffage (17, 18, 21, 22) de manière à maintenir la température de la matière organique entre 50 °C et 60 °C, entre 53 °C et 57 °C ou à approximativement 55 °C.

3. Installation selon la revendication 1 ou 2, **caractérisée en ce que** les organes de brassage (29-38) sont configurés pour être activés de manière à déplacer une énergie thermique à travers une masse de matière organique présente dans le récipient vers le haut à travers lesdites ouvertures (7) pour l'enlèvement de biogaz.

4. Installation selon l'une quelconque des revendications précédentes, **caractérisée en ce que** chaque zone (24-28) comporte au moins deux organes de brassage (29-38) situés côte à côte en vue dans le sens d'alimentation depuis ladite première extrémité à ladite deuxième extrémité de sorte qu'au moins deux rangées (39, 40) d'organes de brassage soient agencées entre ladite première extrémité (2) et ladite deuxième extrémité (4) du récipient.

5. Installation selon la revendication 4, **caractérisée en ce que** l'unité de commande (16) est configurée pour commander lesdits organes de brassage appartenant à l'une et même zone indépendamment l'un de l'autre.

6. Installation selon l'une quelconque des revendications précédentes, **caractérisée en ce que** lesdits organes de brassage peuvent être commandés pour se déplacer autour d'axes de rotation sensiblement verticaux ou verticaux (42).

7. Installation selon la revendication 4 ou 5 et 6, **caractérisée en ce que** le sens de rotation des organes de brassage (29-38) est réversible, et **en ce que** l'unité de commande (16) est configurée pour commander le sens de rotation des organes de brassage appartenant à la même zone indépendamment l'un de l'autre.

8. Installation selon l'une quelconque des revendications précédentes, **caractérisée en ce qu'**elle comprend des moyens (50) pour délimiter au moins partiellement chacune desdites zones (24-28) par rapport à des zones adjacentes.

9. Installation selon la revendication 8, **caractérisée en ce que** lesdits moyens comprennent des cloisons (90) configurées pour délimiter lesdites zones consécutives (24a-28a), et **en ce que** les cloisons sont conçues pour permettre l'alimentation de matière organique depuis une zone à une autre zone en comportant une ou plusieurs ouvertures ou en étant entièrement ou partiellement amovibles depuis une position à laquelle elles séparent des zones adjacentes.

10. Installation selon la revendication 9, **caractérisée en ce que** les cloisons (90) sont conçues pour former des ouvertures entre des zones adjacentes avec une coupe transversale totale de 30 % à 80 %, de 40 % à 60 %, de 50 % à 70 %, de 50 % à 60 % ou d'environ 50 % de la coupe transversale du récipient perpendiculairement au sens d'alimentation depuis la première extrémité (2a) à la deuxième extrémité (4a).

11. Installation selon l'une quelconque des revendications précédentes, **caractérisée en ce que** lesdits organes de brassage (29-38) comprennent des éléments en forme de pale (41) attachés à différents niveaux à un axe (42) pouvant être commandé par ladite unité de commande (16) pour tourner.

12. Installation selon la revendication 11, **caractérisée en ce que** chaque dit axe (42a) est, à l'extrémité inférieure, pourvu d'au moins un élément en forme de pale (94) configuré pour, à la rotation de l'axe, se déplacer comme un râteau le long d'un fond (23a) du récipient de manière à déplacer des particules solides, comme du gravier et des pierres, contenues dans ladite matière organique et déposées sur ce fond.

13. Installation selon la revendication 12, **caractérisée en ce que** chaque dit axe (42a) est agencé à un point central d'une zone rectangulaire, comme un carré, (95) en vue dans le sens d'extension dudit axe, et **en ce que** ledit élément en forme de pale (94) à l'extrémité inférieure de l'axe est conçu pour, à une position de repos, s'étendre depuis l'axe (42a) radialement d'une distance supérieure à la plus courte distance depuis l'axe jusqu'à des pièces (100) définissant ladite zone et est pliable de sorte que, lorsqu'il frappe une dite pièce par le biais d'une rotation de l'axe, il soit replié en stockant une énergie potentielle et à travers l'extrémité extrême de celui-ci se déplace le long de ladite pièce de manière à retourner vers la position de repos lorsque ladite distance jusqu'à ladite pièce augmente.

14. Installation selon l'une quelconque des revendications précédentes, **caractérisée en ce qu'**elle comprend des moyens (80) pour faire recirculer une partie de la matière fermentée atteignant ladite deuxième extrémité (4) du récipient (1) jusqu'à ladite première extrémité (2) pour l'alimenter à nouveau dans le récipient à cette extrémité, ladite partie recirculée étant par exemple de 1 % à 10 % ou de 2 % à 6 % de la matière fermentée atteignant ladite deuxième extrémité.

15. Installation selon l'une quelconque des revendications précédentes, **caractérisée en ce qu'**elle comprend des moyens (14) pour alimenter une matière organique avec un contenu sec de 20 % à 40 %, de 25 % à 35 % ou de 30 % à 35 % dans le récipient.
